# EUROPEAN PATENT SPECIFICATION

(11) **EP 0 665 273 B1**
(45) Date of publication and mention of the grant of the patent: **17.03.1999**
(21) Application number: 95103896.7
(22) Date of filing: 03.09.1992
(51) Int. Cl.: C09C 1/40, C09C 1/00

(54) **New red synthetic mica and ultraviolet ray screening agent comprising the same**
Roter synthetischer Glimmer und diesen enthaltenden gegen Ultraviolettstrahlung abschirmendes Mittel
Mica rouge synthétique et agent faisant écran aux rayons ultraviolets le comprenant

(30) Priority: 04.09.1991 JP 281839/91; 04.09.1991 JP 281840/91
(43) Date of publication of application: 02.08.1995
(62) Divisional of application: 92115097.5
(73) Proprietor: TOPY INDUSTRIES, LIMITED, Tokyo 102 (JP); SHISEIDO COMPANY LIMITED, Chuo-ku Tokyo (JP)
(72) Inventor: Takao, Yuji, Toyohashi-shi, Aichi (JP); Ando, Akitsugu, Toyohashi-shi, Aichi (JP); Kosugi, Tetsusi, Toyohashi-shi, Aichi (JP); Suzuki, Fukuji, Yokohama-shi, Kanagawa (JP); Ohno, Kazuhisa, Yokohama-shi, Kanagawa (JP); Ogawa, Katsuki, Yokohama-shi, Kanagawa (JP)
(74) Representative: Jung, Elisabeth, Dr.

(56) References cited:
- EP-A- 0 220 617
- EP-A- 0 300 047
- EP-A- 0 355 521
- PATENT ABSTRACTS OF JAPAN vol. 15 no. 60 (C-805) ,13 February 1991 & JP-A-02 289417 (NIPPON SHEET GLASS CO.) 29 November 1990,
- DATABASE WPI Section Ch, Week 9113 Derwent Publications Ltd., London, GB; Class A60, AN 91-089809 & JP-A-03 033 179 (TOPY KOGYO K. K.) , 13 February 1991
- PATENT ABSTRACTS OF JAPAN vol. 15 no. 306 (C-856) ,6 August 1991 & JP-A-03 115113 (AGENCY OF IND. SCIENCE AND TECHNOL. ET AL.) 16 May 1991,
- DATABASE WPI Section Ch, Week 8838 Derwent Publications Ltd., London, GB; Class J04, AN 88-267635 & JP-A-63 195 111 (MITSUBISHI MINING CEMENT)
- PATENT ABSTRACTS OF JAPAN vol. 10 no. 120 (C-343) ,6 May 1986 & JP-A-60 246218 (TOPY KOGYO K. K.) 5 December 1985,
- DATABASE WPI Section Ch, Week 9109 Derwent Publications Ltd., London, GB; Class A60, AN 91-060918 & JP-A-03 008 712 (NIPPON SHEET GLASS) , 16 January 1991

## Description

### BACKGROUND OF THE INVENTION

The present invention relates to a method for the manufacturing of new red synthetic mica to be used as base material for paint, ink, coating agent, plastic, film, fiber, glass, and glaze, and also to the use of said red synthetic mica for the production of a composition being capable of effectively screening ultraviolet rays.

Mica powder is used as a filling material for plastics and paint or as pigment for cosmetic products. Furthermore, a mica product having interference color and having a surface being covered with an oxide having a high refractive index, such as titanium oxide, is known as "pearl mica" .

On the other hand, as means for screening ultraviolet rays, methods are known such as a method for screening ultraviolet rays using the scattering effect of the particles, and a method to absorb the ultraviolet ray energy and to convert it to thermal energy. A typical example of the first method is to use titanium oxide, and this is used as an ultraviolet ray scattering agent in cosmetic products, plastics, etc. As the material being representative for the latter-mentioned method, an ultraviolet ray absorbing agent having benzophenone and the like as principal component is known. Furthermore, a large number of ultraviolet ray screening agents have been proposed. For example, there are composite oxides of metals, such as aluminum and zinc (Japanese Patent Laid-Open Publication No. 62-275182), zirconia particle containing cerium (Japanese Patent Laid-Open Publication No. 2-135275), etc. Furthermore, an ultraviolet ray absorbing agent containing mica has also been proposed. For example, there is a product having nickel-containing synthetic mica as the main component (Japanese Patent Laid-Open Publication No. 58-19379), a product having synthetic mica containing Te and the like as the main component (Japanese Patent Laid-Open Publication No. 3-33179), etc. Furthermore, an ultraviolet ray screening pigment has been proposed (Japanese Patent Laid-Open Publication No. 62-187770), which is covered by titanium oxide in such a quantity, that pearl luster due to interference does not virtually appear on mica surface.

On the other hand, mica has been widely used for cosmetic products due to its excellent transparency, luster and high extensibility. In recent years, not only natural mica, but also synthetic mica has been developed as an additive for cosmetic products (Japanese Patent Laid-Open Publication No. 63-241072).

The mica powder belonging to the conventional type has been used in form of scale-like particles having a size of about 1 to 500 µm after being pulverized by a dry or wet type pulverizing method. This conventional type mica powder is disadvantageous as regards the fact, that it can utilize only the heat-resistant and insulating properties of the said mica powder. Due to this reason, an ultraviolet ray absorbing function is provided by giving rise for the obtaining of an interference color through adoption of particle structure covered by titanium oxide, as in the case of the pearl mica described above, or by a method to replace the elements in mica by other elements through synthesis. However, the pearl mica covered by titanium oxide can give rise for the obtaining of an interference color, but it has no transparency being specific for the mica, or titanium oxide has poor resistance to light. The ultraviolet ray absorbing mica which contains specific elements however does not have a sufficient effect as regards its use in order to screen ultraviolet rays.

Regarding the ultraviolet ray screening agent, the ultraviolet ray scattering agent, such as titanium oxide, has an excellent effect in order to screen ultraviolet rays, while it impairs the transparency of mica, because it also scatters the visible rays, and this feature is not suitable for an application which requires transparency. In cases, when it is blended in cosmetic products, white color is exaggerated and gives an unnatural impression. On the other hand, the organic type ultraviolet ray absorbing agent has outstanding ultraviolet ray absorbing potency within a specific wavelength range, but it has poor weatherproof properties and it lacks stability.

Natural mica, muscovite, phlogopite, and biotite are known, and these agents are from grayish white to blackish brown in color. Mica can be synthesized by various methods, and it is known that mica in outstanding white color can be obtained on the base of a synthesis, and that colored synthetic mica can be produced by coordinating "colored" elements in the crystal structure of the above-mentioned white mica.

On the other hand, mica is widely used in cosmetic products, because it has a high transparency, luster and extensibility when it is used. In recent years, not only natural mica, but also synthetic mica has been developed as an additive for cosmetic products. For example, the Japanese Patent Laid-Open Publication No. 63-241072 discloses a synthetic mica powder, in which less fluorine ions are eluted and which has a low surface activity and high oil impregnation properties.

As described above, mica has not been used widely, because it cannot easily be provided in a coloured state, and despite of its excellent properties, only the insulating properties or the leafing properties resulting from its scale-like shape can be utilized. In the fields of paint, plastics, cosmetic products, etc., there is strongly required a material, which is red in color and has an ultraviolet ray screening effect, in order to improve the durability of organic substances. To provide the said red color, however, it is necessary to add a colouring agent, such as an organic dye or iron oxide. Because an organic dies have low resistances towards light, and iron oxide contains particles having a high refractive index, it is likely to cause unevenness and impairs the transparency being important for cosmetic products. On the other hand, an ultra-violet ray scattering agent, such as titanium oxide or an organic type ultraviolet ray absorbing agent are used in cosmetic products, in order to defend the skin against ultraviolet rays. Although an ultraviolet ray scattering agent has an excellent effect in order to screen ultraviolet rays, it also scatters visible rays. This leads to the impairment of transparency and causes the choking and the whitening of the cosmetic products. An organic type ultraviolet ray absorbing agent has an outstanding ultraviolet ray absorbing potency within a specific wavelength range, but it has poor weatherproof properties.

The document EP-A 0 300 047 discloses a synthetic mica powder containing 75 to 99 % fluorine, based on its stoichiometric composition, a process for the production of said mica and its use for the blending of cosmetics. The document "Database WPI, Section Ch, week 8838, Derwent Publications Ltd., London, GB, AN 88-267635" discloses the synthesis of fluorine mica type clay and its use as electric insulating material, catalyst, support or filler. The document "Patent Abstracts of Japan, Vol. 10, No. 120, (C-343), 06.05.1986" mentions a restrictedly swelling synthetic mica and its preparation. The document "Database WPI, Section Ch, week 9109, Derwent Publications Ltd., London, GB, Class A60, AN 91-060918" discloses a synthetic mica having a high whiteness and comprising a chemical composition containing cerium, potassium, magnesium, iron, aluminium, silicon, oxygen and fluorine, and its use for, among others, cosmetical purposes. None of these documents discloses the preparation of *red* mica and the use of the disclosed mica as a component of an UV-ray sreening agent.

### SUMMARY OF THE INVENTION

It is an object of the present invention to provide a method for the manufacturing of a new red synthetic mica, which mica develops red color close to the color of human skin and which effectively screens ultraviolet ray.

It is still another object of the present invention to provide a use for said new red synthetic mica, which mica both is transparent on the one hand, and which mica has bright red color with resistance towards light and which mica therefore is suitable for the use in cosmetic products, in particular, in the foundation.

To attain the above objects, the present inventors have studied with efforts and they have found that, through heat treatment of a synthetic mica having molar number of iron of 0.01 to 3 mols at a specific temperature, a new type of synthetic mica can be obtained, which has a bright red external appearance, which latter was never observed regarding conventional type synthetic mica, and which therefore leads to a very high ultraviolet ray screening potency.

Specifically, the present invention provides a method for the manufacturing of red synthetic mica, which is expressed by the following formula (I)

X_{1/3^{∼} 1} Y_{2^{∼}3} (Z₄ O₁₀) F_{2×0.75^{∼}2×0.99} (I)

which method comprises the steps of:
(a) treating synthetic mica according to the following formula (II)

   X_{1/3^{∼} 1} Y_{2^{∼}3} (Z₄ O₁₀) F₂ (II)

   with an aqueous solution of one or more types of acids or chelating agents, and then
(b) subjecting the product of step (a) to a heat treatment at a temperature in the range of 600 to 1200 °C;
   in which formulae (I) and (II) X represents one or more types of ions selected from the group consisting of Na, K, Li, Ca, Rb and Sr; Y represents one or more types of ions selected from the group consisting of Mg, Fe, Ni, Mn, Al, Li, Co, Ti and Zn; Z represents one or more types of ions selected from the group consisting of Al, Si, Ge, Fe, B, Co and Ti, and at least one of Y and Z represents an iron ion, and whereby the molar number of iron contained in said synthetic mica according to the formulae (I) or (II) being from 0.01 to 3 moles, and when said red synthetic mica according to formula (I) is measured by powder cell method, its color is given by L* = 55 to 75, a* = 5 to 20, and b* = 5 to 20 by the CIE 1976 (L*a*b*) space.

It is another object of the present invention to provide the use of a red synthetic mica, which is expressed by the following formula (I)

X_{1/3^{∼} 1} Y_{2^{∼}3} (Z₄ O₁₀) F_{2×0.75^{∼}2×0.99} (I)

in which formula (I) X represents one or more types of ions selected from the group consisting of Na, K, Li, Ca, Rb and Sr; Y represents one or more types of ions selected from the group consisting of Mg, Fe, Ni, Mn, Al, Li, Co, Ti and Zn; Z represents one or more types of ions selected from the group consisting of Al, Si, Ge, Fe, B, Co and Ti, and at least one of Y and Z represents an iron ion, and whereby the molar number of iron contained in said red synthetic mica being from 0.01 to 3 moles, and when said red synthetic mica is measured by powder cell method, its color is given by L* = 55 to 75, a* = 5 to 20, and b* = 5 to 20 by the CIE 1976 (L*a*b*) space, for the production of a composition being capable of effectively screening ultraviolet rays.

The above-mentioned and the other objects and advantageous features of the invention will become more apparent from the following description.

### BRIEF DESCRIPTION OF THE DRAWING

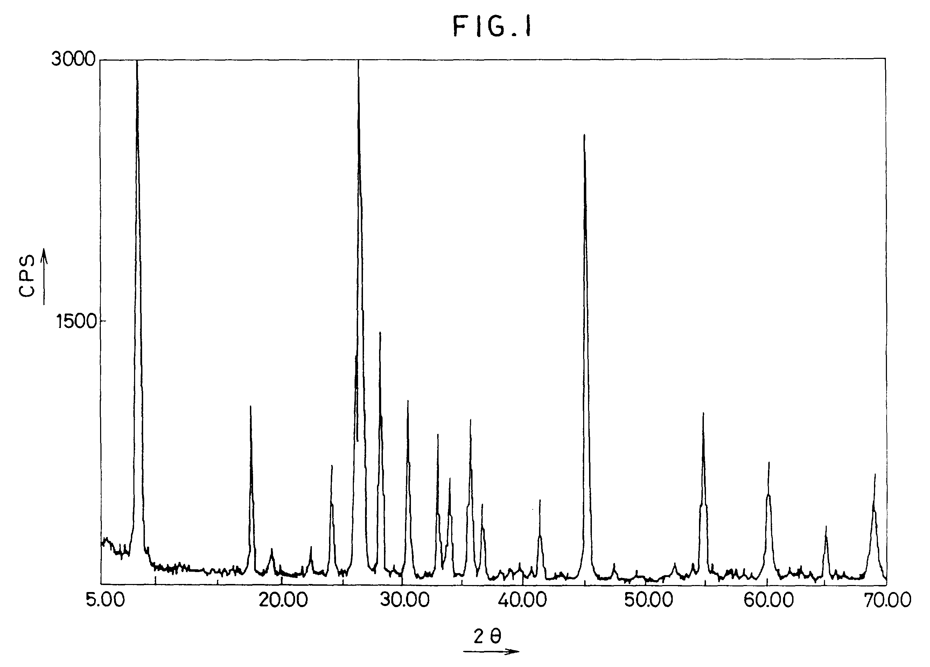

Fig. 1 is an X-ray diffraction chart of composite mica powder being produced in accordance with the method of the present invention as obtained according to the Example 1.

### DETAILED DESCRIPTION OF THE PREFERRED EMBODIMENTS

The red mica being produced in accordance with the method of the present invention must contain Fe, and synthetic mica not containing Fe is not turned to red.

The molar number of Fe contained in the synthetic mica of the present invention is 0.01 to 3 mols, preferably 0.1 to 2 mols, or more preferably, 0.2 to 1 mol. If it is less than 0.01 mol, the red color is too weak. If it exceeds 3 mols, the quality of the mica crystals becomes poor. In the general formula (I) as shown above, Fe is at the octahedron position given by Y, and at the tetrahedron position given by Z. The more the molar number of Fe at the octahedron position is, the more the color of the corresponding mica is turned to red. Therefore, it is necessary as regards the mica being produced according to the method of the present invention, that iron is contained in the crystal structure. Mere attachment on the mica surface does not provide the effect of the mica being produced according to the method of the present invention.

As shown in the above-mentioned general formula of the red mica being produced in accordance with the method of the present invention, the molar number of fluorine in the synthetic mica must be from 75 to 99%, referring to the stoichiometric composition of said synthetic mica, preferably from 80 to 97.5%, and more preferably from 90 to 95%. If it is less than 75%, the mica being produced according to the method of the invention is not turned to synthetic mica. If it exceeds 99%, said mica is not turned to red.

The new red mica being produced in accordance with the method of the present invention is manufactured by treating synthetic mica expressed by the following formula (II):

X_{1/3∼1} Y_{2∼3} (Z₄ O₁₀) F₂ (II)

(where X represents one or more types of ions selected from a group of Na, K, Li, Ca, Rb and Sr, Y represents one or more types of ions selected from a group of Mg, Fe, Ni, Mn, Al, Li, Co, Ti and Zn, Z represents one or more types of ions selected from a group of Al, Si, Ge, Fe, B, Co, and Ti, and at least one of Y and Z represents an iron ion), and the mica being produced according to the method of the invention is produced from a synthetic mica having a molar number of Fe contained in synthetic mica at 0.01 to 3 mols through a heat treatment at a temperature being in the range between 600 to 1200°C.

The synthetic mica serving as raw material, as expressed by the above formula (II), can be synthesized by the hydrothermal method, the solid phase reaction method, the fused synthetic method, etc. The mica thus synthesized is pulverized to powder having a size of about 0.1 to 100 µm through a wet type or a dry type pulverization. After treating the mica powder with an aqueous solution of one or more types of acids or chelating agents, it is processed by a heat treatment at a temperature being in the range between 600 to 1200°C, and the red scale-like mica being produced in accordance with the method of the present invention can be produced.

As regards the acids in order to treat the mica powder, inorganic acids or organic acids may be used. As regards the inorganic acids, there are available hydrochloric acid, nitric acid, sulfuric acid, chloric acid, perchloric acid, periodic acid, bromic acid, phosphoric acid, boric acid and carbonic acid. As regards the organic acids, there are available carboxylic acids, such as formic acid, acetic acid, acrylic acid, benzoic acid, oxalic acid, malonic acid, succinic acid, glutaric acid, adipic acid, pimelic acid and phthalic acid, oxycarboxylic acids, such as lactic acid, malic acid, tartaric acid, citric acid, etc., and amino acids, such as glycine, alanine, valine, leucine, thyrosin, threonine, serine, proline, tryptophan, methionine, cystine, thyroxine, asparagine, glutamic acid, lysine, and arginine.

As regards chelating agents, there are available ethylenediaminetetraacetic acid, nitrilotriacetic acid, 1,2-diaminocyclohexanetetraacetic acid, N-oxyethylethylenediamine-triacetic acid, ethyleneglycol-bis-tetraacetic acid, ethylenediaminetetrapropionic acid, etc.

The time and temperature for the treating of the said mica powder by means of acids or chelating agents may be properly selected according to the types and the respective concentration of the acids or the chelating agents. In general, the treating time may be in the range between several minutes to several days, and the treating temperature may be in the range between 0 and 100°C. The concentration of the treating aqueous solution is preferably in the range between 0.05 and 10 mols/liter. For example, when the treating is performed at 20 °C using 0.1N hydrochloric acid, it is preferable that the processing time is adjusted in the range between 30 minutes and 5 hours.

As regards the processing methods, the method using an agitator, or other known methods using aeration and dipping may be used.

The heat treatment temperature is selected in the range between 600 and 1200°C, or preferably between 900 and 1100°C. If it is lower than 600°C, the mica is not turned to red. If it exceeds 1200°C, the said mica is not turned to red and the mica is decomposed.

The respective time for the heat treatment may be adjusted in an interval ranging between several seconds and several days and may further be properly selected according to the heat treatment temperature. For example, when fluorine phlogopite containing 0.2 mol of Fe at the octahedron position is used as the raw material, it is preferable to treat at a temperature of 1000°C for 0.5 to 10 hours.

As heat treatment atmosphere, an oxidyzing atmosphere, a reducing atmosphere, an argon gas atmosphere, an N₂ atmosphere, an ammonia gas atmosphere or a vacuum may be used alone or in combination, and this may be selected properly according to the application, function, etc. of the synthetic mica being produced according to the method of the invention.

As heat treatment equipment, any type of the furnaces already known, such as a gas furnace, an electric furnace, a rotary kiln, a batch furnace, etc. may be used.

The red mica expressed by the formula (I) being produced in accordance with the method of the present invention can be blended and mixed with cosmetic pigment, coloring pigment, ultraviolet ray scattering agent and ultraviolet ray absorbing agent, in order to use said mica for the preparation of an prepare ultraviolet ray agent.

As regards the cosmetic pigments to be used in the present invention, there are available talc, kaolin, mica, calcium carbonate, magnesium carbonate, magnesium silicate, silicic anhydride, barium sulfate, nylon powder, polyethylene powder, polystyrene powder, etc. As regards the coloring pigment, there are available iron oxide, ultramarine blue, chromium oxide, Prussian blue, carbon black, etc. As regards the ultraviolet ray scattering agents, there are available titanium oxide, titanium oxide ultra-fine particles, zinc oxide, etc. As regards the ultraviolet ray absorbing agents, there are available benzophenone type or benzotriazole type ultraviolet ray absorbing agents.

It is preferable to blend the red mica produced according to the method of the invention and given by the formula (I) by 1 to 100%, and the blending ratio should be properly selected according to each application, desired property, etc.

Referring an invention relating to the present invention, the Japanese Patent Laid-Open Publication No. 2-289417 discloses an ultraviolet ray absorbing synthetic mica, which contains Fe (converted to Fe₂O₃) by 2 to 18 weight %, bivalent Fe (converted to FeO) by less than 0.8 weight %, and in which weight the ratio of FeO-converted value to the sum of the FeO-converted value and the Fe₂O₃-converted value is lower than or equal to 0.1, i.e. (FeO/FeO+ Fe₂O₃ ≦ 0.1).

However, the synthetic mica as described in the above-mentioned patent publication is a synthetic mica having a high whitening degree, and there has never been provided any description of a type of synthetic mica, which is colored in red, as the mica produced according to the method of the present invention.

According to Nobutoshi Daimon (Kogyo Kagaku Zasshi, Vol. 55, No. 11), it is described that Ni-containing mica has a greenish yellow colour, Mn-containing mica has a brown colour, Co-containing mica has a pink or blue colour, Fe-containing mica has a greenish black or brown (dark brown) colour, and there is no description of a mica having red colour. The results of the colour determination of the Fe-containing mica having a brown colour reveal that the value of a*, which is used as a measure for reddening, is about -1 to +1, and this value is different from the value of a*=5 to 20 corresponding to the case of the red scale-like mica being produced according to the method of the present invention.

It has been found in the present invention, that mica in transparent and bright red color can be obtained by placing a specific element at a specific position and by setting the molar number of fluorine and the molar number of the Fe ions within specific ranges. Such effects are not anticipated by the prior art as described above.

Furthermore, a detailed description will be provided regarding the features of the method according to the present invention in connection with the examples, however, the present invention will not be limited to these examples.

### (Example 1)

A mixture containing potassium silicofluoride by 24.4 g, magnesium oxide by 22.3 g, aluminum oxide by 11.3 g, silicon dioxide by 33.2 g and iron oxide by 8.8 g was mixed for 30 minutes in a V type mixer. Then, this mixture was placed in an alumina crucible, melted for 30 minutes at a temperature of 1450°C in an electric furnace and subsequently cooled down in the furnace. Thus, 95 g of iron-containing synthetic mica was prepared. After pulverizing this mica in a ball mill for 48 hours, 90 g of this iron-containing synthetic mica powder was processed at normal temperature for 48 hours in 1 liter of an aqueous solution of 0.05N hydrochloric acid while agitating. Then it was dehydrated, rinsed and dried. 80g of this dried powder were placed in an alumina crucible. Through heat treatment at 1000°C for 10 hours in an electric furnace, 75 g of the synthetic mica, produced in accordance with the method of the present invention, were obtained. The synthetic mica powder thus obtained had a red colour. When measured by the X-ray diffraction system RAD-B (Rigaku Denki-Sha), the X-ray diffraction pattern agreed well with that of the synthetic mica as given in Fig.1. When the iron-content was analyzed by the ESCA-method, it was confirmed, that iron was included in the crystal structure.

Regarding the synthetic mica thus prepared in accordance with the method of the invention, L*a*b*, the UV transmittance and the molar numbers of the included iron and fluorine were measured by the following methods. The results are summarized in Table 1.

### (Measuring methods)

(1) Measurement of L*a*b*: 1 g of the specimen was placed in the powder cell of a color-difference meter (CR-200; Minolta) and measured.
(2) Measurement of the UV transmittance: The specimen was added to castor oil at a concentration of 20%. After mixing by a 3-roll mill, it was coated on a quartz plate in a thickness of 10 µm. UV transmittance at 300 nm was measured using a UV spectrophotometer (Shimadzu).
(3) Measurement of the molar numbers of the included iron and fluorine in the synthetic mica powder: Iron was measured by the atomic absorption spectrophotometry method, and fluorine was measured by the absorption spectrophotometry method.

### (Example 2)

Red synthetic mica, produced in accordance with the method of the present invention was obtained in the quantity as shown in Table 1 by the same procedure as described in the Example 1, except that 24.7 g of potassium silicofluoride, 27.1 g of magnesium oxide, 5.7 g of aluminum oxide, 33.6 g of silicon dioxide and 8.9 g of iron oxide were mixed for 30 minutes by means of a V type mixer. When the X-ray diffraction pattern was measured by the same procedure as described in the Example 1, it agreed well with that corresponding to the synthetic mica. Utilizing the same procedure as described in the Example 1, it was confirmed that iron was included in the respective crystal structure. As regards the mica thus prepared, L*a*b*, the UV transmittance, and the molar numbers of the included iron and the fluorine were measured by the same procedure as already described in the Example 1. The results are summarized in Table 1.

### (Example 3)

The red synthetic mica produced in accordance with the method of the present invention was obtained in the quantity as shown in Table 1 by means of using the same procedure as described in the Example 1, except that the heat treatment temperature was adjusted to 700°C in the Example 1. When measured by the same procedure as in the Example 1, the X-ray diffraction pattern agreed well with that corresponding to synthetic mica. Utilizing the same procedure as described in the Example 1, it was confirmed that iron was included in the crystal structure. Regarding the mica thus prepared, L*a*b*, the UV transmittance and the molar numbers of the included iron and the fluorine were measured by means of the same procedure as described in the Example 1. The results are summarized in Table 1.

### (Example 4)

The red synthetic mica produced in accordance with the method of the present invention was obtained in the quantity as shown in Table 1 by means of the same procedure as described in the Example 1, except that the heat treatment temperature was adjusted to 1200°C and the heat treatment time was adjusted to 5 minutes in the Example 1. When measured by means of the same procedure as described in the Example 1, the X-ray diffraction pattern agreed well with that corresponding to synthetic mica. Utilizing the same procedure as described in the Example 1, it was confirmed, that iron was included in the crystal structure. Regarding the mica thus obtained, L*a*b*, the UV transmittance and the molar numbers of the included iron and the included fluorine were measured as described in Example 1. The results are given in Table 1.

### (Example 5)

The red synthetic mica produced in accordance with the method of the present invention was obtained in the quantity as shown in Table 1 by means of the same procedure as described in the Example 1, except that a 10% citric acid aqueous solution was used instead of the 0.05N hydrochloric acid as used in the Example 1. When measured by means of the same procedure as described in the Example 1, the X-ray diffraction pattern agreed well with that corresponding to synthetic mica. Utilizing the same procedure as described in the Example 1, it was confirmed that iron was included in the crystal structure. As regards the mica thus obtained, L*a*b*, the UV transmittance and the molar numbers of the included iron and the included fluorine were measured as described in Example 1. The results are given in Table 1.

### (Comparative example 1)

25.5 g of potassium silicofluoride, 28.0 g of magnesium oxide, 11.8 g of aluminum oxide, and 34.8 g of silicon dioxide were mixed for 30 minutes in a V type mixer, and synthetic mica which did not contain iron was obtained by the same procedure as described in the Example 1. Regarding the mica thus prepared, L*a*b*, the UV transmittance, and the molar numbers of the iron and the fluorine as included were measured by means of the same procedure as described in the Example 1. The results are summarized in Table 1.

### (Comparative example 2)

Synthetic mica was prepared by means of the same procedure as described in the Example 1, except that the heat treatment was not performed. As regards the mica thus prepared, L*a*b*, the UV transmittance and the molar numbers of the included iron and the included fluorine were measured by means of the same procedure as described in the Example 1. The results are summarized in Table 1.

### (Comparative example 3)

Synthetic mica was prepared by means of the same procedure described as in the Example 1, except that the heat treatment temperature was adjusted to 600°C. As regards the mica thus prepared, L*a*b*, the UV transmittance and the molar numbers of the included iron and the included fluorine were measured by means of the same procedure as described in the Example 1. The results are summarized in Table 1.

### (Comparative example 4)

Synthetic mica was prepared by means of the same procedure as described in the Example 1, except that the heat treatment temperature was adjusted to 1300°C.

As regards the mica thus prepared, L*a*b*, the UV transmittance and the molar numbers of the included iron and the included fluorine were measured by means of the same procedure as described in the Example 1. The results are summarized in Table 1.

**(Table 1)**

| Results of the evaluation | | | | | | | | | |
|---|---|---|---|---|---|---|---|---|---|
| | Molar number of Fe contained | F molar number | Heat treatment temperature ( °C ) | External appearance | Yield (g) | L*a*b* | | | UV transmittance (%) |
| | | | | | | L* | a* | b* | |
| Example 1 | 0.3 | 1.9 | 1000 | Red | 75 | 65 | 11.0 | 10.2 | 58 |
| Example 2 | 0.4 | 1.9 | 1000 | Red | 73 | 68 | 8.5 | 7.3 | 61 |
| Example 3 | 0.3 | 1.94 | 700 | Red | 75 | 70 | 10.2 | 9.9 | 60 |
| Example 4 | 0.3 | 1.9 | 1200 | Red | 75 | 64 | 9.0 | 8.8 | 62 |
| Example 5 | 0.3 | 1.9 | 1000 | Red | 74 | 66 | 10.8 | 9.5 | 60 |
| Comparative Example 1 | 0 | 1.9 | 1000 | White | 70 | 90 | -1.5 | 0.0 | 99 |
| Comparative Example 2 | 0.3 | 2 | None | Whitish brown | 75 | 77 | 0.2 | 7.3 | 58 |
| Comparative Example 3 | 0.3 | 2 | 600 | Whitish Yellow | 75 | 75 | 1.1 | 7.5 | 60 |
| Comparative Example 4 | 0.3 | 1.4 | 1300 | Whitish Yellow | 74 | 75 | 1.5 | 7.3 | 90 |

As described above, it is possible according to the method in accordance with the present invention to obtain new synthetic mica, which has transparent property and light-resistant red colour, which has not been found in the field of any conventional type mica products. This is attained by means of coordinating a specific element at a specific position and by adjusting the molar number of fluorine and the molar number of iron ions thus being within specific ranges. Because the synthetic mica produced in accordance with the method of the present invention has an excellent ultraviolet ray screening effect, it can be mixed and used in cosmetic products, plastics, paint, etc. as an ultraviolet ray screening agent and without using any colouring agent. Furthermore, there is no problem corresponding to the deterioration of the red color due to the light. Red scale-like mica being produced in accordance with the method of the present invention has a color being closely related to that of human skin and furthermore it has a transparent and bright red color, and it is especially useful for the application in cosmetic products, particularly in the foundation.

## Claims

1. A method for the manufacturing of red synthetic mica having an iron content being in the range of between 0.01 and 3 moles, which is expressed by the following formula (I)
X_{1/3^{∼} 1} Y_{2^{∼}3} (Z₄ O₁₀) F_{2×0.75^{∼}2×0.99} (I)
and which method comprises the steps of:
(a) treating synthetic mica according to the following formula (II)
X_{1/3^{∼} 1} Y_{2^{∼}3} (Z₄ O₁₀) F₂ (II)
with an aqueous solution of one or more types of acids or chelating agents, and then
(b) subjecting the product of step (a) to a heat treatment at a temperature in the range of 600 to 1200 °C;
in which formulae (I) and (II) X represents one or more types of ions selected from the group consisting of Na, K, Li, Ca, Rb and Sr; Y represents one or more types of ions selected from the group consisting of Mg, Fe, Ni, Mn, Al, Li, Co, Ti and Zn; Z represents one or more types of ions selected from the group consisting of Al, Si, Ge, Fe, B, Co and Ti, and at least one of Y and Z represents an iron ion, and whereby the molar number of iron contained in said synthetic mica according to the formulae (I) or (II) being from 0.01 to 3 moles, and, when said red synthetic mica according to formula (I) is measured by powder cell method, its color is given by L* = 55 to 75, a* = 5 to 20, and b* = 5 to 20 by the CIE 1976 (L*a*b*) space.

2. Use of a red synthetic mica having an iron content being in the range of between 0.01 and 3 moles, which is expressed by the following formula (I)
X_{1/3^{∼} 1} Y_{2^{∼}3} (Z₄ O₁₀) F_{2×0.75^{∼}2×0.99} (I)
in which formula (I) X represents one or more types of ions selected from the group consisting of Na, K, Li, Ca, Rb and Sr; Y represents one or more types of ions selected from the group consisting of Mg, Fe, Ni, Mn, Al, Li, Co, Ti and Zn; Z represents one or more types of ions selected from the group consisting of Al, Si, Ge, Fe, B, Co and Ti, and at least one of Y and Z represents an iron ion, and whereby the molar number of iron contained in said red synthetic mica being from 0.01 to 3 moles, and, when said red synthetic mica is measured by powder cell method, its color is given by L* = 55 to 75, a* = 5 to 20, and b* = 5 to 20 by the CIE 1976 (L*a*b*) space,
for the production of a composition being capable of effectively screening ultraviolet rays.

## Patentansprüche

1. Eine Methode zur Herstellung von rotem synthetischen Glimmer mit einem Eisengehalt im Bereich von 0,01 bis 3 Mol, welcher besagte Glimmer mit Hilfe der nachfolgend wiedergegebenen, allgemeinen Formel (I) beschrieben werden kann:
X_{1/3^{∼}} ₁ Y_{2^{∼}3} (Z₄ O₁₀) F_{2x0,75^{∼} 2x0,99} (I),
und welche Methode die nachfolgend beschriebenen Schritte umfasst:
(a) die Behandlung von synthetischem Glimmer, welcher der nachfolgend wiedergegebenen, allgemeinen Formel (II) entspricht,
X_{1/3^{∼} 1} Y_{2 ^{∼} 3} (Z₄ O₁₀) F₂ (II)
mit einer wäßrigen, eine oder mehrere Säurearten oder Chelatbildner enthaltenden Lösung, und anschließend
(b) die Unterwerfung des nach dem vorstehenden Schritt (a) erhaltenen Produktes unter eine Hitzebehandlung bei einer Temperatur im Bereich von 600 bis 1200 °C;
in welchen vorstehend wiedergegebenen, allgemeinen Formeln (I) und (II) die allgemeine Bezeichnung X eine oder mehrere Ionenarten darstellt, die aus der Gruppe ausgewählt worden sind, welche aus Na, K, Li, Ca, Rb und Sr besteht; in welchen die allgemeine Bezeichnung Y eine oder mehrere Ionenarten darstellt, die aus der Gruppe ausgewählt worden sind, die aus Mg, Fe, Ni, Mn, Al, Li, Co, Ti und Zn besteht; in welchen die allgemeine Bezeichnung Z eine oder mehrere Ionenarten darstellt, die aus der Gruppe ausgewählt worden sind, die aus Al, Si, Ge, Fe, B, Co und Ti besteht, und wobei mindestens eine der beiden allgemeinen Bezeichnungen Y oder Z ein Eisenion darstellt, und wobei sich die Stoffmenge des in den beiden synthetischen Glimmerarten entsprechend den beiden vorstehend wiedergegebenen, allgemeinen Formeln (I) und (II) enthaltenen Eisens im Bereich von 0,01 bis 3 Mol befindet, und mit der zusätzlichen Maßgabe, daß die Farbe des besagten, roten synthetischen Glimmers entsprechend der vorstehend wiedergegebenen, allgemeinen Formel (I), wenn sie mit Hilfe der sogenannten Pulverzellmethode (engl.: *"powder cell method"*) gemessen wird, auf der Grundlage des Farbenraumes mit der Bezeichnung CIE 1976 (L*a*b*) durch die folgenden Zahlenwerte definiert ist:
L* = 55 bis 75, a* = 5 bis 20 und b* = 5 bis 20.

2. Verwendung eines roten, synthetischen Glimmers mit einem Eisengehalt im Bereich von 0,01 bis 3 Mol, welcher besagte Glimmer mit Hilfe der nachfolgend wiedergegebenen, allgemeinen Formel (I) beschrieben werden kann:
X_{1/3 ^{∼} 1} Y_{2^{∼}3} (Z₄ O₁₀) F_{2x0,75 ^{∼} 2x0,99} (I),
in welcher vorstehend wiedergegebenen, allgemeinen Formel (I) die allgemeine Bezeichnung X eine oder mehrere Ionenarten darstellt, die aus der Gruppe ausgewählt worden sind, welche aus Na, K, Li, Ca, Rb und Sr besteht; in welchen die allgemeine Bezeichnung Y eine oder mehrere Ionenarten darstellt, die aus der Gruppe ausgewählt worden sind, die aus Mg, Fe, Ni, Mn, Al, Li, Co, Ti und Zn besteht; in welchen die allgemeine Bezeichnung Z eine oder mehrere Ionenarten darstellt, die aus der Gruppe ausgewählt worden sind, die aus Al, Si, Ge, Fe, B, Co und Ti besteht, und wobei mindestens eine der beiden allgemeinen Bezeichnungen Y oder Z ein Eisenion darstellt, und wobei sich die Stoffmenge des in dem besagten, roten synthetischen Glimmer enthaltenen Eisens im Bereich von 0,01 bis 3 Mol befindet, und mit der zusätzlichen Maßgabe, daß die Farbe des besagten, roten synthetischen Glimmers entsprechend der vorstehend wiedergegebenen, allgemeinen Formel (I), wenn sie mit Hilfe der sogenannten Pulverzellmethode (engl.: *"powder cell method"*) gemessen wird, auf der Grundlage des Farbenraumes mit der Bezeichnung CIE 1976 (L*a*b*) durch die folgenden Zahlenwerte definiert ist: L* = 55 bis 75, a* = 5 bis 20 und b* = 5 bis 20,
für die Herstellung einer Zusammensetzung, die zur wirksamen Abschirmung gegen ultraviolette Strahlen geeignet ist.

## Revendications

1. Procédé de fabrication de mica rouge synthétique ayant une teneur en fer dans le domaine de 0,01 à 3 mol, qui est exprimé par la formule (I) suivante :
X_{1/3 ^{∼} 1} Y_{2 ^{∼} 3} (Z₄ O₁₀) F_{2x0,75 ^{∼} 2x0,99} (I)
et lequel procédé comprend les étapes de :
(a) traitement de mica synthétique selon la formule (II) suivante :
X_{1/3 ^{∼} 1} Y_{2 ^{∼} 3} (Z₄ O₁₀) F₂ (II)
avec une solution aqueuse d'un ou plusieurs types d'acides ou d'agents chélatants, puis
(b) soumission du produit de l'étape (a) à un traitement thermique à une température dans le domaine de 600 à 1 200°C ;
formules (I) et (II) dans lesquelles X représente un ou plusieurs types d'ions choisis dans le groupe consistant en Na, K, Li, Ca, Rb et Sr ; Y représente un ou plusieurs types d'ions choisis dans le groupe consistant en Mg, Fe, Ni, Mn, Al, Li, Co, T et Zn ; Z représente un ou plusieurs types d'ions choisis dans le groupe consistant en Al, Si, Ge, Fe, B, Co et Ti, et parmi Y et Z l'un au moins représente un ion fer, de sorte que le nombre molaire de fer contenu dans ledit mica synthétique selon les formules (I) ou (II) est de 0,01 à 3 mol, et, lorsque ledit mica rouge synthétique selon la formule (I) est mesuré par le procédé à cellule de poudre, sa couleur est donnée par L* = 55 à 75, a* = 5 à 20, et b* = 5 à 20 par l'espace (L*a*b*) de la CIE 1976.

2. Utilisation d'un mica rouge synthétique ayant une teneur en fer comprise dans le domaine de 0,01 à 3 mol, qui est exprimé par la formule (I) suivante :
X_{1/3 ^{∼} 1} Y_{2 ^{∼} 3} (Z₄ O₁₀) F_{2x0,75 ^{∼} 2x0,99} (I)
formule (I) dans laquelle X représente un ou plusieurs types d'ions choisis dans le groupe consistant en Na, K, Li, Ca, Rb et Sr ; Y représente un ou plusieurs types d'ions choisis dans le groupe consistant en Mg, Fe, Ni, Mn, Al, Li, Co, Ti et Zn ; Z représente un ou plusieurs types d'ions choisis dans le groupe consistant en Al, Si, Ge, Fe, B, Co et Ti, et parmi Y et Z l'un au moins représente un ion fer, de sorte que le nombre molaire de fer contenu dans ledit mica rouge synthétique est de 0,01 à 3 mol et, lorsque ledit mica rouge synthétique est mesuré par le procédé à cellule de poudre, sa couleur est donnée par L* = 55 à 75, a* = 5 à 20, et b* = 5 à 20 par l'espace (L*a*b*) de la CIE 1976,
pour la production d'une composition capable de filtrer efficacement les rayonnements ultraviolets.
